(19) Europäisches Patentamt
European Patent Office
Office européen des brevets

(11) Veröffentlichungsnummer : **0 009 648**
**B1**

(12) # EUROPÄISCHE PATENTSCHRIFT

(45) Veröffentlichungstag der Patentschrift : **11.08.82**

(51) Int. Cl.³ : **C 09 F 1/04**, C 08 G 65/32, B 01 F 17/52

(21) Anmeldenummer : **79103298.0**

(22) Anmeldetag : **05.09.79**

(54) **Grenzflächenaktive Verbindungen auf Basis natürlicher Harzsäuren und ihre Verwendung.**

(30) Priorität : **15.09.78 DE 2840113**

(43) Veröffentlichungstag der Anmeldung : **16.04.80 (Patentblatt 80/08)**

(45) Bekanntmachung des Hinweises auf die Patenterteilung : **11.08.82 Patentblatt 82/32**

(84) Benannte Vertragsstaaten : **BE CH DE FR GB IT NL**

(56) Entgegenhaltungen :
**DE A 1 769 331**
**FR A 946 189**
**FR A 1 008 635**
**US A 2 950 272**

(73) Patentinhaber : **HOECHST AKTIENGESELLSCHAFT**
**Postfach 80 03 20**
**D-6230 Frankfurt/Main 80 (DE)**

(72) Erfinder : **Cuntze, Ulrich, Dr.**
**Chesapeake Drive 4331**
**Charlotte, N.C. 28208 (US)**
Erfinder : **Uhrig, Heinz**
**Feldbergstrasse 59**
**D-6374 Steinbach/Taunus (DE)**

**EP 0 009 648 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Grenzflächenaktive Verbindungen auf Basis natürlicher Harzsäuren und ihre Verwendung

Umsetzungsprodukte von Harzalkoholen, Harzaminen und Harzsäuren, ihren Salzen, Anhydriden, Estern, Amiden oder Halogeniden mit Polyglykoläthern oder Äthylenoxid, die auf 1 Mol des Ausgangsstoffes mindestens einen Polyglykolrest mit 8 oder mehr Äthylenoxidgruppen enthalten, eignen sich zum Waschen, Netzen, Reinigen, Egalisieren, Emulgieren sowie als Korrosionsschutzmittel (Ullmanns Encyklopädie der technischen Chemie, 3. Aufl., Band 8, Seite 408 ff).

Veresterungsprodukte von Harzsäuren und Harzsäuremaleinaten mit Polyalkoholen, wie z.B. Glyzerin, Pentaerythrit und Sorbit, finden außer auf dem Lackgebiet Anwendung bei der Herstellung von Druckfarben und Beim Harzen von Papierleimen sowie als Emulgatoren für die Kaltpolymerisation von Butadien-Styrol-Mischungen bei der Kautschukherstellung (Ullmann, a.a.O., Seite 412, und J. Scheiber, Chemie und Technologie der künstlichen Harze (1943), Seite 560). Ferner werden für die Herstellung von Farben, Lacken und plastischen Massen Kolophonium-Phenol-Verbindungen verwendet, die man durch Anlagerung von Phenol, Kresol oder Naphthol an Naturharzsäuren erhält (DE-A 581 956, 582 846, 652 602 und 536 170). Die DE-A 689 392 betrifft die Herstellung von Gerbstoffen und Textilhilfsmittel durch Umsetzungen von Naturharzen, vorwiegend Kolophonium, mit aromatischen Hydroxyverbindungen in Gegenwart von Schwefelsäure als Katalysator und Kondensation mit Formaldehyd und Natriumsulfit.

Aus der FR-A 946 189 ist es bekannt, Kolophonium-Polyglykoläther mit sulfonierenden Mitteln zu behandeln. Die Produkte eignen sich als Hilfsmittel bei der Textilherstellung, -färbung und -bearbeitung, für Leder, Öle, Fette, Wachse, Papier und dergleichen.

Aus der DE-A 1 769 331 sind Umsetzungsprodukte von Äthylenoxyd mit Naturharzen, insbesondere Tallölharz, bekannt, die unter anderem bei der Herstellung von Phosphorsäure nach dem Naßverfahren zugesetzt werden können, um die Schaumbildung zu unterdrücken.

Die Verwendung von Tallölharzsäure-Oxalkylaten als nichtionische Tenside ist auch aus der US-A 2 950 272 bekannt. Gegebenenfalls können diese Verbindungen als Endgruppe einen weiteren Acylrest der Tallölharzsäure tragen.

Aus der FR-A 1 008 635 ist es bekannt, die freien Hydroxygruppen oder andere reaktionsfähige Gruppen oder Atome in Oxalkylaten von Harzsäuren und ihren Derivaten umzusetzen, beispielsweise mit Schwefelsäure. Als Harzsäurederivate werden unter anderem hydrierte Abietinsäure und Kondensationsprodukte des Kolophoniums mit Maleinsäure sowie ein Kondensationsprodukt aus Glycerin-maleinat und Kolophonium genannt. Die Produkte sind oberflächenaktiv und eignen sich als Dispergiermittel, u.a. für Pigmente, sowie als Färbereihilfsmittel.

Gegenstand der Erfindung sind Verbindungen der Formel

$$A[(X-O)_x-Y-Z]_m$$

in der

A für den Rest einer natürlichen Harzsäure, ihrer Disproportionierungs- oder Hydrierungsprodukte, eines Harzsäuremaleinats, einer durch Reaktion mit einer cycloaliphatischen, araliphatischen oder aromatischen halogenabspaltenden Verbindung oder einer phenolischen Verbindung modifizierten Harzsäure oder ein Veresterungsprodukt einer gegebenenfalls wie vorstehend definierten modifizierten Harzsäure mit einem mehrwertigen Alkohol steht,

X für gleiche oder verschiedene Gruppen der Formel $-CH_2-CH_2-$ und $-CH_2-CH(CH_3)-$ steht,

Y gleiche oder verschiedene Reste der Formel $-C_nH_{2n}-$ bedeutet, worin n 2 oder 3 ist,

Z für gleiche oder verschiedene Reste der Formeln $-OH$ oder $-O-CO-CH_2-CH(SO_3M)-COOM$ steht, worin M ein Kation bedeutet, wobei mindestens ein Rest Z von $-OH$ verschieden ist,

x eine Zahl von 1 bis 100 ist, und

m eine ganze Zahl von 1 bis 5 bedeutet.

Der Harzrest A leitet sich vorzugsweise von folgenden Verbindungen ab:

a) natürliche Harzsäuren und/oder ihre Disproportionierungsprodukte, wie sie in handelsüblichen Kolophoniumarten vorliegen oder daraus gewonnen werden,

b) Harzsäuremaleinate, wie sie aus den unter a) genannten Harzsäuren durch Umsetzung mit Maleinsäureanhydrid und anschließender Veresterung der Anhydridgruppe erhalten werden,

c) Harzalkohole, wie sie aus den unter a) genannten Harzsäuren durch Reduktion, insbesondere Hydrierung, entstehen,

d) modifizierte Harzsäuren, wie sie durch Addition an oder Kondensation mit aromatischen Hydroxyverbindungen bzw. halogenabspaltenden aromatischen Zwischenprodukten aus den unter a) genannten Harzsäuren gewonnen werden,

e) Veresterungsprodukte und/oder deren Gemische, wie man sie durch Veresterung aus 1 Mol eines 2- bis 5-wertigen Alkohols mit 1 bis 4, vorzugsweise 1 bis 2 Mol einer Harzsäure bzw. einer modifizierten Harzsäure, wie sie unter a) und d) genannt werden, erhält.

Y ist vorzugsweise $-CH_2-CH_2-$ oder $-CH_2-\underset{\substack{|\\ CH_3}}{CH}-$ :

Z steht vorzugsweise für $-O-CO-CH_2-\underset{\displaystyle SO_3M}{CH}-COOM$

Der Index x ist vorzugsweise 2 bis 18, m ist vorzugsweise 2 bis 3 und M vorzugsweise Wasserstoff, ein Alkalimetall, ein Äquivalent Erdalkalimetall, eine Ammoniumgruppe, insbesondere eine sich von einem niedermolekularen Alkylamin- oder Alkylolamin ableitende Ammoniumgruppe.

Gegenstand der Erfindung ist weiterhin die Verwendung dieser Verbindungen als grenzflächenaktive Mittel, insbesondere als Dispergiermittel für die Feinverteilung und Stabilisierung von Feststoffen, sowie als Netz-, Emulgier-, Egalisier- und Färbereihilfsmittel.

Die erfindungsgemäßen Verbindungen werden erhalten, indem man natürliche Harzsäuren, disproportionierte Harzsäuren oder mit aromatischen Hydroxyverbindungen bzw. halogenabspaltenden Cycloalkyl- und insbesondere Aralkyl- und Arylverbindungen modifizierte Harzsäuren sowie deren Veresterungsprodukte mit mehrwertigen Alkoholen, Harzalkohole, Harzmaleinate und/oder Gemische dieser genannten Harzkörper mit Äthylenoxid und/oder Propylenoxid oxalkyliert und die so erhaltenen Alkylenoxidaddukte mit Maleinsäureanhydrid zu den entsprechenden Halbestern umsetzt und an die Maleinsäurehalbester dann Alkali- oder Erdalkalisulfite oder -hydrogensulfite addiert.

Als Ausgangsprodukte eignen sich natürliche Harzsäuren wie Abietinsäure, Dehydroabietinsäure, Dihydroabietinsäure, Tetrahydroabietinsäure, Levopimarsäure, Dextropimarsäure und Isodextopimarsäure, wie sie in handelsüblichen Kolophoniumarten vorliegen, sowie disproportionierte, hydrierte und dimerisierte Harzsäuren, Harzalkohole wie Abietyl- und Hydroabietylalkohol (vorwiegend bestehend aus Dehydroabietyl-, Dihydroabietyl- und Tetrahydroabietylalkohol), Harzsäuremaleinate, deren Anhydridgruppe z.B. mit Glykol, Diglykol, Glyzerin, 1,1,1-Trimethylolpropan, Pentaerythrit oder auch einwertigen Alkoholen verestert ist.

Als weitere Ausgangsprodukte eignen sich Harz-Phenol-Verbindungen, wie sie durch Addition z.B. von Phenol, Kresol, o-Kresolacetat, Salicylsäure, Guajacol, Bisphenol A, und β-Naphthol an natürliche Harzsäuren bzw. handelsübliche Kolophoniumarten in Gegenwart von stark sauren oder säureabspaltenden Katalysatoren, wie z.B. Bortrifluorid, Chlorwasserstoff, Zinntetrachlorid, Aluminiumchlorid oder Mineralsäuren bei Temperaturen zwischen 20 und 120 °C in organischem Medium gewonnen werden, wobei auf 1 Mol Harzsäure 0,5 bis 0,8, vorzugsweise 0,7 Mol der genannten phenole eingesetzt werden.

Weiterhin eignen sich modifizierte Naturharzsäuren, wie sie durch Umsetzung von Naturharzsäuren mit halogenabspaltenden araliphatischen oder aromatischen Verbindungen, z.B. Benzylchlorid, Bischlormethylbenzol, Chlormethyltoluol, Benzalchlorid, Chlormethylnaphthalin, 2-, 3- oder 4-Chlorphenol, 5-Chlor-2-hydroxytoluol, 2-Chlor-5-hydroxy-1,3-xylol, 4-Chlordiphenyl, 1- oder 2-Chlornaphthalin, 1-Chlor-2-naphthol und 2-Chlor-1-naphthol oder cycloaliphatischen Verbindungen wie Cyclohexylchlorid in Gegenwart eines Katalysators, wie etwa 0,2 % Zinkchlorid, bei Temperaturen zwischen 100 bis 220 °C, vorzugsweise 150 bis 210 °C, gewonnen werden, wobei auf 1 Mol Harzsäure 0,5 bis 1,0, vorzugsweise 0,7 bis 0,8 Mol der genannten Chlorkohlenwasserstoffe umgesetzt werden.

Hier und im folgenden beziehen sich Prozentangaben auf das Gewicht, wenn nichts anderes angegeben ist.

Als Vertreter der mehrwertigen Alkohole zur Veresterung der Harzsäuren seien beispielsweise genannt: Glykol, Glyzerin, 1,2,4-Butantriol, Butandiol-(1,4), 1,1,1-Trimethylolpropan, Pentaerythrit, 2,4-Dihydroxy-3-methylol-pentan, Hexantriol, Sorbit, Anhydrosorbit, Hexit oder Mannit. Die Veresterung der Harzsäuren mit den mehrwertigen Alkoholen erfolgt im Molverhältnis 1 : 1 bis 4 : 1, vorzugsweise 1 : 1 bis 2 : 1, nach bekannten Veresterungsverfahren bei 180 bis 300 °C, vorzugsweise bei 200 bis 270 °C, gegebenenfalls unter Zusatz eines Schlepplittels wie eines aromatischen Kohlenwasserstoffes oder Chlorkohlenwasserstoffes. Als Katalysatoren können z.B. Benzolsulfonsäure, p-Toluolsulfonsäure, Borsäure, Zinnpulver oder Schwefelsäure verwendet werden.

Die Oxalkylierung der Harzsäuren, Harzsäuremaleinate, Harzalkohole und Harzsäureester erfolgt nach bekannten methoden, vorzugsweise mit einem Alkalihydroxid oder -alkoxid als Katalysator bei 100 bis 200 °C, bevorzugt bei 140 bis 170 °C. Die Menge Alkylenoxid wird so bemessen, daß pro Carboxygruppe der Harzsäuren und Harzsäuremaleinate sowie pro Hydroxygruppe der Harzalkohole je 1 bis 50, vorzugsweise 2 bis 18 Mol, und auf die freien Hydroxygruppen der Harzsäureester je 1 bis 50, vorzugsweise 3,5 bis 9 Mol Alkylenoxid angelagert werden. Als Alkalihydroxid eignen sich Kaliumhydroxid oder bevorzugt Natriumhydroxid, als Alkalialkoxid Natriummethylat oder -äthylat; die Konzentration soll bevorzugt 0,05 bis 1,0 %, bezogen auf Harzsäure, Harzsäuremaleinat, Harzalkohol oder Harzester, bei Beginn der Oxalkylierung sein. Die Oxalkylierung kann drucklos oder in Druckgefäßen mit Propylenoxid oder bevorzugt Äthylenoxid oder Mischungen von beiden durchgeführt werden, wobei das Alkylenoxid gasförmig oder flüssig zugeführt werden kann. Der Arbeitsdruck beträgt 1 bis 10, vorzugsweise 2 bis 8 bar.

Im folgenden werden einige zweckmäßige Verfahren zur Einführung der anionaktiven Gruppe(n) näher beschrieben.

Die Reaktion der Oxalkylate mit Maleinsäureanhydrid zu den Maleinsäurehalbestern erfolgt durch Mischen der Komponenten und Verrühren bei 20 bis 100 °C, bevorzugt 40 bis 80 °C, in Anwesenheit von Alkalihydroxiden, deren Konzentration 0,1 bis 1,0 %, bezogen auf die Gesamtmischung, betragen soll. Da

Maleinsäureanhydrid zur Sublimation neigt, ist es vorteilhaft, in Druckgefässen unter einem Überdruck von 0,2 bis 1,0 bar Stickstoff oder Luft zu arbeiten und für kräftige Durchmischung zu sorgen, da zu Beginn der Reaktion das geschmolzene Maleinsäureanhydrid mit den Oxalkylaten schlecht mischbar ist. Die Menge des Maleinsäureanhydrids kann so bemessen werden, daß alle Oxalkylat-Hydroxyendgruppen oder nur ein Teil hiervon, mindestens jedoch eine, umgesetzt werden.

Die Überführung dieser Maleinsäurehalbester-Verbindungen in die entsprechenden Sulfobernsteinsäurehalbester erfolgt nach Zugabe von wäßrigen Lösungen von Sulfiten oder Hydrogensulfiten. Auf jede Maleinsäurehalbestergruppe werden 1,0 bis 1,5, bevorzugt 1,05 bis 1,1 Äquivalente Alkali- oder Erdalkalisulfit, -hydrogensulfit bzw. -pyrosulfit eingesetzt. Die Sulfite sind dabei besonders geeignet, da hierbei die Di-Salze der Sulfobernsteinsäurehalbester entstehen. Die zugesetzte Wassermenge kann 50 bis 85 Gew.-%, bezogen auf die gesamte Lösung bzw. Mischung, betragen und ist von der Löslichkeit der Sulfobernsteinsäurehalbestersalze und der Viskosität der Lösung abhängig. Die Reaktionstemperaturen betragen 20 bis 100 °C, bevorzugt 40 bis 80 °C.

Die erfindungsgemäßen Verbindungen und deren Gemische sind in Wasser klar löslich und zeigen nach dem Ross-Miles Test (DIN 53 902) nur eine geringe Schaumneigung bei gleichzeitiger Herabsetzung der Oberflächenspannung nach der Ringabreißmethode (DIN 53 914) bis zu optimal 30 $10^{-3}$ N/m. Sie netzen außerdem Baumwollgewebe nach der Tauchnetzmethode (DIN 63 901) innerhalb 30 bis 240 Sekunden, sind gegen starkes Alkali sowie gegen starke Säuren unter üblichen Anwendungsbedingungen von Tensiden beständig und besitzen als Reinigungsmittel ein ausgezeichnetes Schmutztragevermögen. Ihre Eigenfarbe ist als sprühgetrocknetes Pulver sowie als wäßrige Lösung sehr hell bis nahezu farblos, wodurch beim Färben von salzbildenden Fasern keine Anschmutzeffekte entstehen.

Die erfindungsgemäßen Verbindungen eignen sich hervorragend als Dispergier-, Netz- und Verteilungsmittel, z.B. für die Herstellung hochpigmentierter und gut fließfähiger Pigmentpräparationen, bevorzugt als Dispergiermittel für Farbstoffdispersionen, besonders von Dispersionsfarbstoffen, für die Herstellung von Dispersionen optischer Aufheller, für die Formulierung von Pflanzenschutz- und Schädlingsbekämpfungsmitteln sowie als Emulgiermittel für die Herstellung von Carrieremulsionen, insbesondere von Chlorbenzolen, Chlorphenolen, Hydroxydiphenylen, Diphenyl, Phenylphenolen, aromatischen Carbonsäureestern und Alkoholen sowie Naphthalin und dessen Derivaten, und als Egalisier- und Färbereihilfsmittel zum Färben von natürlichem und synthetischem Fasermaterial, wie Baumwolle, Wolle, Cellulose, Zellwolle, Celluloseacetat und -triacetat, Polyester, Polyamid und Polyacrylnitril, oder von Fasermaterialien, die diese Stoffe enthalten.

Die Überlegenheit der erfindungsgemäßen Substanzen gegenüber bekannten anionischen Dispergiermitteln, wie Ligninsulfonaten oder Formaldehydkondensationsprodukten auf der Basis Naphthalinsulfonsäure, Alkylnaphthalinsulfonsäure und Phenolsulfonsäure sowie Kresol-Formaldehyd oder Phenol-Formaldehyd und Natriumsulfit, besteht darin, daß sie bei deutlich verbessertem Dispergierverhalten im wäßrigen Anwendungsbereich zusätzlich stark netzende Eigenschaften aufweisen. Die Produkte können biologisch leicht abgebaut werden.

Die erfindungsgemäßen Verbindungen können wahlweise in Kombination mit nichtionogenen oder anderen anionaktiven Verbindungen, Gerüstsubstanzen und anderen Zusatz- und Hilfsstoffen in den Dispergier- und Emulgierformulierungen zur Anwendung kommen.

Die nachfolgenden Beispiele dienen zur näheren Erläuterung der Erfindung.

## Beispiele

In den folgenden Beispiel A-L wird die herstellung der erfindungsgemäßen Verbindungen beschrieben. Teile und Prozentangaben beziehen sich auf das Gewicht, Druckangaben sind « Überdruck », sofern nichts anderes angegeben ist.

### Verbindung A

a) Herstellung des Harzsäureoxalkylates

302 Tl. Kolophonium werden nach Zugabe von 2,2 Tl. Natriummethylat in einem Druckgefäß unter Rühren und Zuführen von 440 Tl. Äthylenoxid bei 150 bis 160 °C unter Aufrechterhaltung eines Druckes von 7 bis 9 bar oxalkyliert. Wenn das ganze Äthylenoxid aufgedrückt ist, wird 1 Stunde bei 150 bis 160 °C nachgerührt. Das erhaltene Harzsäureaddukt enthält 10 Mol Äthylenoxid.

b) Herstellung des Sulfobernsteinsäurehalbesters

742 Tl. Harzsäureaddukt werden mit 98 Tl. Maleinsäureanhydrid unter Rühren bei 50 bis 80 °C versetzt ; anschließend nach Verdünnen mit Wasser werden 132,3 bis 138,6 Tl. Natriumsulfit (als wäßrige Lösung) bei 40 bis 80 °C innerhalb von 15 bis 120 Minuten zugerührt, bis der Ansatz klar löslich geworden ist. Anschließend wird 1 Stunde nachgerührt. Die Menge des zugesetzten Wassers kann zwischen 50 und 85 Gewichtsprozent der Endlösung betragen.

### Verbindung B

a) Herstellung des Harzalkoholoxalkylates

288 Tl. Harzalkohol (technischer Hydroabietylalkohol der Firma Hercules) werden nach Zugabe von 1 Tl. Kaliumhydroxid in einem Druckgefäß unter Rühren und Zuführen von 364 Tl. Äthylenoxid bei 150 bis 160 °C unter Aufrechterhaltung eines Druckes von etwa 1,5 bis 2 bar oxalkyliert. Wenn das ganze Äthylenoxid aufgedrückt ist, wird 1 Stunde bei 150 bis 160 °C nachgerührt. Das erhaltene Harzalkoholaddukt enthält 8,3 Mol Äthylenoxid.

b) Herstellung des Sulfobernsteinsäurehalbesters

652 Tl. Harzalkoholaddukt werden mit 98 Tl. Maleinsäureanhydrid unter Rühren bei 50 bis 80 °C verestert und anschließend nach Verdünnen mit Wasser werden 132,3 bis 138,6 Tl. Natriumsulfit (als wäßrige Lösung) bei 40 bis 80° innerhalb 15 bis 120 Minuten zugerührt, bis der Ansatz klar wasserlöslich geworden ist. Anschließend wird 1 Stunde nachgerührt. Die Menge des zugesetzten Wassers kann zwischen 50 und 85 Gewichtsprozent der Endlösung betragen.

### Verbindung C

a) Herstellung des Harzsäureglyzerinesters

302 Tl. disproportioniertes Kolophonium werden in Gegenwart von 4 Tl. Zinnpulver mit 92 Tl. Glyzerin in einem geeigneten Rührgefäß unter Abdestillieren des Reaktionswassers innerhalb 8 bis 10 Stunden bei 230 bis 250 °C bei gleichzeitigem Durchleiten von Stickstoff bis zu einer Säurezahl (SZ) (DIN 53 183) von 25 verestert.

b) Herstellung des Harzsäureglyzerinester-Oxalkylates

376 Tl. Harzsäureglyzerinester werden nach Zugabe von 2,7 Tl. Natriummethylat in einem Druckgefäß unter Rühren und Zuführen von 308 Tl. Äthylenoxid bei 150 bis 160 °C unter Aufrechterhaltung eines Druckes zwischen 2 bis 8 bar oxalkyliert. Wenn das ganze Äthylenoxid aufgedrückt ist, wird 1 Stunde bei 150 bis 160 °C nachgerührt. Das erhaltene Harzsäureglyzerinesteraddukt enthält 7 Mol Äthylenoxid.

c) Herstellung des Di-sulfobernsteinsäurehalbesters

690 Tl. Harzsäureglyzerinester-Oxalkylat werden mit 196 Tl. Maleinsäureanhydrid unter Rühren bei 50 bis 80 °C verestert ; anschließend nach Verdünnen mit Wasser werden 264,6 bis 277,2 Tl. Natriumsulfit (als wäßrige Lösung) bei 40 bis 80 °C innerhalb 15 bis 120 Minuten zugerührt, bis der Ansatz klar wasserlöslich geworden ist. Anschließend wird 1 Stunde nachgerührt. Die Menge des zugesetzten Wassers kann zwischen 50 und 85 Gewichtsprozent der Endlösung betragen. Durch Sprühtrocknung erhält man ein elektrolytarmes weißes Pulver.

### Verbindung D

a) Herstellung des oxäthylierten Harzsäureglyzerinesters

302 Tl. Kolophonium werden entsprechend Beispiel C a) mit 92 Tl. Glyzerin verestert und gemäß Beispiel C b) mit 792 Tl. Äthylenoxid oxalkyliert. Das erhaltene Harzsäureesteraddukt enthält 18 Mol Äthylenoxid.

b) Herstellung des Di-Sulfobernsteinsäurehalbesters

1 094 Tl. Harzsäureglyzerinesteraddukt werden entsprechend Beispiel C c) mit 196 Tl. Maleinsäureanhydrid und mit 264,6 bis 277,2 Tl. Natriumsulfit umgesetzt. Nach der Sprühtrocknung erhält man ein leicht voluminöses weißes Pulver.

### Verbindung E

a) Herstellung des Harzsäurepentaerythritesters

468 Tl. Kolophonium werden in Gegenwart von 6,7 Tl. Borsäure mit 204 Tl. Pentaerythrit entsprechend Beispiel C a) bis zu einer SZ von 14,8 verestert.

b) Herstellung des Harzsäureester-Oxalkylates

428 Tl. Harzsäurepentaerythritester werden nach Zugabe von 3,1 Tl. Natriummethylat entsprechend Beispiel C b) mit 308 Tl. Äthylenoxid umgesetzt. Das erhaltene Harzsäureesteraddukt enthält 7 Mol Äthylenoxid.

c) Herstellung des Tri-Sulfobernsteinsäurehalbesters

736 tl. Harzsäureester-Oxalkylat werden gemäß Beispiel C c) mit 294 Tl. Maleinsäureanhydrid und 378 Tl. Natriumsulfit zum Tri-Sulfobernsteinsäurehalbester umgesetzt. Durch Zerstäubungstrocknung erhält man ein fast salzfreies weißes Pulver.

Verbindung F

a) Herstellung des Di-Harzsäure-mono-sorbitesters

604 Tl. Kolophonium werden in Gegenwart von 8 Tl. Zinnpulver mit 182 Tl. Sorbit nach Beispiel C a) verestert. SZ : 18,7.

b) Herstellung des Harzsäuresorbit-Oxalkylates

750 Tl. Harzsäuresorbitester a) werden nach Zugabe von 5,4 Tl. Natriummethylat gemäß Beispiel C b) mit 660 Tl. Äthylenoxid umgesetzt. Das erhaltene Harzsäuresorbitaddukt enthält 15 Mol Äthylenoxid.

c) Herstellung des Di-Sulfobernsteinsäurehalbesters

705 Tl. Harzsäuresorbitester-Oxalkylat werden entsprechend Beispiel C c) mit 98 Tl. Maleinsäureanhydrid und 132,3 bis 138,6 Tl. Natriumsulfit zum Di-Sulfobernsteinsäurehalbester umgesetzt.

Verbindung G

a) Herstellung des Tri-Sulfobernsteinsäurehalbesters aus F b)

705 Tl. Harzsäuresorbitester von Beispiel F b) werden mit 147 Tl. Maleinsäureanhydrid und 198,5 bis 207,9 Tl. Natriumsulfiut zum Tri-Sulfobernsteinsäurehalbester umgesetzt.

Verbindung H

a) Herstellung des Harz-Oxalkylates

486 Tl. Kolophoniummaleinatharz (Schmp. n. Kapillarmethode (DIN 53 181) 110-118 °C ; Dichte (20 °C) 1,11 ; SZ 10-20 ; Jodfarbzahl (DIN 6162) 30) werden nach Zugabe von 1 Tl. Natriummethylat entsprechend Beispiel A a) mit 440 Tl. Äthylenoxid umgesetzt. Das erhaltene Harzaddukt enthält 10 Mol Äthylenoxid.

b) Herstellung des Sulfobernsteinsäurehalbesters

926 Tl. Harzaddukt werden gemäß Beispiel A b) mit 98 Tl. Maleinsäureanhydrid und 132,3 bis 138,6 Tl. Natriumsulfit zum Sulfobernsteinsäurehalbester umgesetzt.

Verbindung I

a) Herstellung der Harz-Phenol-Verbindung

173,8 Tl. einer 25 %igen Lösung von Bortrifluorid in Phenol gibt man bei 10 °C innerhalb 4 Stunden zu einer Lösung von 604 Tl. Kolophonium in 800 Tl. Tetrachlormethan und läßt 14 Stunden bei 15 bis 18 °C nachrühren. Nach Aufarbeitung erhält man 630 Tl. eines klaren Harzes mit einer SZ von etwa 114 und einem Schmp. von ca. 105 °C.

b) Herstellung des Harz-Oxalkylates

367 Tl. des unter a) hergestellen modifizierten Harzes werden nach Zugabe von 1,5 Tl. Natriumhydroxid in einem Druckgefäß unter Rühren und Zuführen von 378,4 Tl. Äthylenoxid bei 150° bis 170 °C unter Aufrechterhaltung eines Druckes zwischen 1,5 und 2,5 bar oxäthyliert. Wenn das ganze Äthylenoxid aufgedrückt ist, wird eine Stunde bei 150 bis 160 °C nachgerührt. Das erhaltene Harz-Oxalkylat enthält 8,6 Mol Äthylenoxid.

c) Herstellung des Di-sulfobernsteinsäurehalbesters

745,4 Tl. Harzaddukt b) werden mit 196 Tl. Maleinsäureanhydrid unter Rühren bei 50 bis 80 °C verestert ; anschließend, nach Verdünnen mit Wasser, werden 264,6 bis 277,2 Tl. Natriumsulfit (als wäßrige Lösung) bei 40 bis 80 °C innerhalb 15 bis 120 minuten zugerührt und eine Stunde nachgerührt, nachdem der Ansatz klar wasserlöslich geworden ist. Die Menge des zugesetzten Wassers kann zwischen 50 und 85 Gewichtsprozent der Endlösung betragen. Durch Sprühtrocknung erhält man ein elektrolytarmes hellgelbes Pulver.

Verbindung K

a) Herstellung der Harz-Kresol-Verbindung

200 Tl. einer 25 %igen Lösung von Bortrifluorid in techn. Kresol gibt man bei 10 °C innerhalb 4 Stunden zu einer Lösung von 604 Tl. Kolophonium in 800 Tl. Tetrachlormethan und läßt 14 Stunden bei 15 bis 18 °C nachrühren. Nach Aufarbeitung erhält man 767 Tl. eines klaren Harzes mit der SZ von 117 und einem Schmp. von 105 bis 110 °C.

b) Herstellung des Harz-Kresol-Glyzerinesters

377 Tl. Harz-Kresol-Verbindung a) werden in Gegenwart von 5 Tl. Zinnpulver mit 92 Tl. Glyzerin in

einem geeigneten Rührgefäß unter Abdestillieren des Reaktionswassers innerhalb von 8 bis 10 Stunden bei 230 bis 250 °C bei gleichzeitigem Durchleiten von Stickstoff bis zu einer SZ von etwa 25 verestert.

c) Herstellung des Harz-Kresol-Glycerinesteroxalkylates

451 Tl. Harz-Kresol-Glycerinester b) werden gemäß Beispeil I b) nach Zugabe von 3,3 Tl. Natriummethylat mit 440 Tl. Äthylenoxid oxalkyliert. Das erhaltene Harzesteraddukt enthält 10 Mol Äthylenoxid.

d) Herstellung des Di-sulfobernsteinsäurehalbesters

891 Tl. Harzglycerinesteraddukt c) werden entsprechend Beispiel I c) mit 196 Tl. Maleinsäureanhydrid und 264,6 bis 277,2 Tl. Natriumsulfit umgesetzt. Durch anschließend Zerstäubungstrocknung erhält man fast elektrolytfreies hellgelbes Pulver.

Verbindung L

a) Herstellung des Harz-Benzyl-Glyzerinesters

302 Tl. Kolophonium werden mit 80 Tl. Benzylchlorid gemischt und langsam auf 100 °C erhitzt, wobei die Chlorwasserstoffentwicklung beginnt. Die Chlorwasserstoffentwicklung kann durch Zugabe von 0,2 Tl. Zinkchlorid beschleunigt werden. Sowie die Chlorwasserstoffentwicklung nachläßt, wird die Temperatur auf 200 °C erhöht und 1 Stunde bei 200 bis 210 °C gehalten, bis das Umsetzungsprodukt praktisch halogenfrei ist. Nach Abkühlung auf etwa 100 °C und Zugabe von 30 Tl. Glyzerin und 100 Tl. Xylol wird auf 240 bis 250 °C geheizt und bei dieser Temperatur etwa 4 Stunden gehalten. Nach Entfernung der flüchtigen Stoffe erhält man ein helles Harz mit der SZ 30 und einem Erweichungspunkt von 120 bis 125 °C.

b) 384 Tl. Harz-Benzyl-Glycerinester a) werden gemäß Beispiel I b) nach Zugabe von 2,8 Tl. Natriummethylat mit 440 Tl. Äthylenoxid oxäthyliert. Das erhaltene Harzglyzerinaddukt enthält 10 Mol Äthylenoxid.

c) Herstellung des Di-Sulfobernsteinsäurehalbesters

824 Tl. Harzglyzerinesteraddukt b) werden entsprechend Beispiel I c) mit 196 Tl. Maleinsäureanhydrid und 264,6 bis 277,2 Tl. Natriumsulfit umgesetzt. Nach der Trocknung im Zerstäubungsapparat erhält man ein hellgelbes Pulver mit geringem Elektrolytgehalt.

Prüfbeispiele

Für den Vergleich der Dispergiereigenschaften der Verbindungen A bis L werden die in der DE-A 21 32 403 beschriebenen Prüfverfahren und Farbstoffe benutzt.

Bei den Prüfverfahren 1 bis 3 werden verschiedene lösliche Farbstoffe in Gegenwart wachsender Mengen des zu prüfenden Tensids bei drei pH-Werten ausgefällt und ihre Feinverteilung durch die jeweilige oberflächenaktive Substanz geprüft. Die in der Tabelle angeführten Zahlen bedeuten das Verhältnis von Farbstoff : Tensid, bei dem der Farbstoff ohne Rückstand dispergiert werden kann (d.h. je kleiner die angegebene Zahl, um so größer die Dispergierfähigkeit). Im Prüfverfahren 4 wird ein mittelschwer dispergierbarer Dispersionsfarbstoff in einer 1 l-Rührwerksmühle feinverteilt ; die hierfür nötige Mahlzeit in Studen ist in der Tabelle 1 angegeben. Die hierbei entstehende Dispersion wird bei 50 °C gelagert und wiederholt geprüft.

In der Tabelle ist die geprüfte Zeit (in Tagen) angegeben, in der sich die Dispersion noch nicht verschlechtert hat. Als Maß für das Färbeverhalten bei 130 °C wurde Polyester nach bekannten Verfahren gefärbt. Das Ergebnis wird in Tabelle 1 durch die Ziffern 1 bis 5 festgelegt. Es bedeuten :

1 kein Färbevermögen ;
2 schwach ;
3 brauchbar ;
4 gut ;
5 sehr gut.

Im Vergleich zu den Dispergiereigenschaften der Tabelle 1 werden in der Tabelle 2 die oberflächenaktiven Eigenschaften nach folgenden DIN-Normen angegeben :

Netzkraft DIN 53 901 ; Schaumvermögen : DIN 53 902 ; Oberflächenspannung : DIN 53 914.

Die Beurteilung des Schaumvermögens wurde nach der Einteilung

0 nicht schäumend ;
1 schwach ;
2 schwach-mittel ;
3 mittel ;
4 stark

vorgenommen.

Dabei wurden die Ergebnisse der erfindungsgemäßen Verbindungen A bis L den Ergebnissen der bekannten Dispergiermittel X bis Z gegenübergestellt. Beim Dispergiermittel X handelt es sich um ein handelsübliches Kondensationsprodukt aus Naphthalin-2-sulfonsäure mit Formaldehyd, das mit Natriumcarbonat neutralisiert wurde (BIOS Final Report 762, Seite 70 Mitte ; Ausgabe der Hobard Publishing Company, Washington DC, USA), während das Dispergiermittel Y nach der im FIAT Final Report 1013 beschriebenen Vorschrift aus Kresol, 1-Naphthol-6-sulfonsäure, Natriumsulfit und Formaldehyd hergestellt wurde. Z repräsentiert die FR-A 949 189 und wird wie folgt aus dem nach Beispiel Aa) erhaltenen Harzsäure-Oxalkylat hergestellt :

742 Tl. Harzsäureaddukt werden mit 101,8 bis 106,7 Tl. Amidosulfosäure und 6 Tl. Harnstoff vermischt und 7,5 Stunden bei 122 bis 125 °C unter Stickstoffatmosphäre gerührt, bis ein hellgelbes, sehr zähes Reaktionsprodukt vorliegt. Zur Überführung in das Natriumsalz werden 843,8 bis 848,7 Tl des Ammoniumsalzes mit 600 Tl. Wasser und 1 000 Tl. Natronlauge (24 %ig) versetzt und unter Einleiten von Stickstoff und Rühren so lange auf 70 °C erwärmt, bis das gesamte Ammoniak ausgetrieben ist. Das wäßrige Endprodukt enthält 26 % Natriumsalz.

(Siehe die Tabelle 1, Seite 9)

Tabelle 1

| Verbindung | Prüfverfahren | | | | Lagerstabilität | Polyesterfärbung |
|---|---|---|---|---|---|---|
| | 1 (pH 2-5) | 2 (pH 7) | 3 (pH 9-13) | 4 Mahlzeit (h) | in Tagen | bei 130°C |
| X | >5 | 0,125 | 0,5 | 5 | 1 | 3 - 4 |
| Y | 1,5 | 0,125 | >2 | 4 | >42 | 4 - 5 |
| Z | 1 | 0,125 | 1 | 3 | <1 | 5 |
| A | 1 | 0,125 | 0,5 | 1 | >21 | 4 |
| B | 0,5 | 0,125 | 0,5 | 1 | <1 | 5 |
| C | 1 | 0,125 | 1 | 1 | >42 | 5 |
| D' | 1 | 0,125 | 0,5 | 1 | >42 | 5 |
| E | 1 | 0,125 | 0,5 | 1 | >21 | 5 |
| F | 1 | 0,125 | 1 | 1 | >42 | 5 |
| G | 1 | 0,125 | 1 | 1 | >42 | 5 |
| H | 1 | 0,125 | 1 | 1 | <1 | 5 |
| I | 0,5 | 0,065 | 0,5 | 1 | >42 | 4 |
| K | 0,5 | 0,065 | 0,5 | 1 | >42 | 5 |
| L | 1 | 0,125 | 0,5 | 1 | >42 | 5 |

Tabelle 2

| Verb. | Netzkraft (sec.) 20°C | Netzkraft (sec.) 70°C | Schaumvermögen C = 2 g/l | Oberflächenspannung ($10^{-3}$ N/m) C = 2 g/l | Jodfarbzahl C = 2 g/l |
|---|---|---|---|---|---|
| X | - | - | 0 | 71,30 | 1 |
| Y | - | - | 2 | 58,50 | 50 |
| Z | - | 120 | 1 | 39,00 | 1 |
| A | 310 | 54 | 0 | 43,80 | 1 |
| B | 189 | 65 | 1 | 39,68 | 1 |
| C | - | 199 | 0 | 39,50 | 1 |
| D | 443 | 150 | 0 | 42,80 | 1 |
| E | - | 226 | 0 - 1 | 46,20 | 1 |
| F | - | - | 1 | 41,60 | 1 |
| G | - | - | 1 | 33,60 | 1 |
| H | - | 238 | 0 - 1 | 45,00 | 1 |
| I | 272 | 57 | 1 | 41,88 | 1 |
| K | - | 260 | 1 | 47,48 | 1 |
| L | - | 200 | 0 - 1 | 44,80 | 1 |

## Anwendungsbeispiele

Neben diesem allgemeinen Vergleich der Dispergier- und Netzeigenschaften sollen die folgenden Beispiele das breite Anwendungsspektrum der erfindungsgemäßen Substanzen erläutern :

### Beispiel 1

50 Teile des Dispersionsfarbstoffes 2-Methyl-3-äthoxy-carbonyl-6-morpholino-10-oxopyrazolo [2,3-b] benzo [d,e]-isochinolin werden mit 40 Teilen der Verbindung C und 110 Teilen Wasser in einer Rührwerksmühle 4 Stunden bis zur Feinverteilung gemahlen. Nach Zugabe von 50 Teilen Wasser erhält man einen 20 %igen Farbteig mit sehr guter Feinverteilung, der allen coloristischen Anforderungen, besonders beim Färben von Polyester, Polyester/Wolle- und Polyester/Zellwolle-Mischgespinsten genügt.

Ähnlich gute Ergebnisse erhält man mit den Verbindungen A, D, G, K und L sowie mit Mischungen dieser Substanzen mit den Verbindungen B, F, H und I im Verhältnis 9 : 1 bis 1 : 9, vorzugsweise 3 : 1 bis 1 : 3.

### Beispiel 2

50 Teile des Dispersionsfarbstoffes 4-(2-Chlor-4-nitrophenylazo)-3-methyl-N-äthyl-N-β-cyanäthylanilin werden gemäß Beispiel 1 mit 40 Teilen der Verbindung C und 110 Teilen Wasser in 3 Stunden bis zur Feinverteilung gemahlen. Die erhaltene Dispersion wird durch Zugabe von 10 Teilen eines Naphthalinsulfosäure-Formaldehyd-Kondensationsproduktes und weiterem Stellmittel auf 30 % Farbstoffgehalt eingestellt und in einem Sprühtrockner zu Pulver getrocknet. Das Farbstoffpulver erfüllt alle coloristischen und färbetechnischen Anforderungen. Gleiche Ergebnisse erhält man mit den Verbindungen D, E, I, K und L.

### Beispiel 3

88,1 Teile des in Beispiel 1 genannten Dispersionsfarbstoffes werden mit 26 Teilen der Verbindung C, 44 Teilen Glykol und 43,4 Teilen Wasser in einer Rührwerksmühle 4 Stunden bis zur Feinverteilung gemahlen. Der Teig wird mit Wasser auf eine konzentration von 40 % Farbstoff eingestellt. Der erhaltene hochprozentige Farbteig ist aufgrund seiner guten Fließfähigkeit und Stabilität zur Herstellung von Druckpasten für den Transferdruck hervorragend geeignet.

### Beispiel 4

72,7 Teile des Dispersionsfarbstoffes 2-(4-Brom-3-hydroxy-chinolyl)-1,3-indandion werden entsprechend Beispiel 3 mit 20,6 Teilen der Verbindung C, 36,4 Teilen Glykol und 73,1 Teilen Wasser feinverteilt und durch Zugabe von Wasser auf einen Gehalt von 35 % Farbstoff eingestellt. Der erhaltenen Farbteig besitzt eine gute Fließfähigkeit und entspricht den Anforderungen für den Transferdruck.

### Beispiel 5

60 Teile des Pigmentes Pigment Yellow 12 (Colour Index Nr. 21090) werden in einer Lösung aus 16,4 Teilen der Verbindung D in 57,5 Teilen Wasser unter Zusatz von 600 Teilen Sili-Quarzit-Perlen (1 bis 2 mm Durchmesser) als Mahlkörper 2 Stunden in einer 1-Liter-Laborperlmühle gemahlen. Nach Zumischung von 20 Teilen Glykol saugt man die Mahlkörper über ein Sieb ab und erhält eine gießbare Pigmentdispersion mit einem Pigmentgehalt von 40 Gewichtsprozent.

Ähnlich gute Ergebnisse erhält man mit den Verbindungen C, K und L.

### Beispiel 6

160 Teile des im Beispiel 1 der DE-A 14 44 014 beschriebenen Aufhellers werden mit 10 Teilen der Verbindung K und 160 Teilen Wasser in einer Perlmühle 2 Stunden bis zur Feinvertelung gemahlen und die Dispersion wird auf einen Gehalt von 20 % Aufheller verdünnt. Man erhält eine stabile nichtabsetzende dünnflüssige Dispersion. Die gleichen Ergebnisse erhält man mit den Verbindungen C, D, E und L.

### Beispiel 7

100 Teile des Pflanzenschutzmittels 2-Carbomethoxy-amino-benzimidazol werden mit 10 Teilen der Verbindung D, 4 Teilen des Natriumsalzes einer polymerisierten Alkylsulfonsäure, 10 Teilen eines Fettalkoholadduktes auf Basis eines Blockpolymerisates aus Polypropylenglykol, an das Äthylenoxid angelagert wurde, und 76 Teile Wasser 2 Stunden bis zur Feinverteilung in einer 1-Liter-Rührwerksmühle gemahlen. Nach Abtrennung der Mahlkörper erhält man eine sehr stabile Dispersion mit einer guten Schwebefähigkeit ohne Bodensatz.

Beispiel 8

73,9 Teile Methylnaphthalin werden mit 49,3 Teilen der 53 %igen Einstellung nach Beispiel A homogen verrührt; das Konzentrat wird mit Wasser auf 1 Liter aufgefüllt. Man erhält eine feindisperse Carrieremulsion, die in ihrer üblichen Verdünnung 1 : 10 eine hervorragende Stabilität besitzt und über längere Zeit verwendet werden kann.

**Ansprüche**

1. Verbindungen der Formel

$$A[(X—O)_x—Y—Z]_m$$

in der

A für den Rest einer natürlichen Harzsäure, ihrer Disproportionierungs- oder Hydrierungsprodukte, eines Harzsäuremaleinats, einer durch Reaktion mit einer cycloaliphatischen, araliphatischen oder aromatischen halogenabspaltenden Verbindung oder einer phenolischen Verbindung modifizierten Harzsäure oder ein Veresterungsprodukt einer gegebenenfalls wie vorstehend definierten modifizierten Harzsäure mit einem mehrwertigen Alkohol steht,

X für gleiche oder verschiedene Gruppen der Formel $—CH_2—CH_2—$ und $—CH_2—CH(CH_3)—$ steht,

Y gleiche oder verschiedene Reste der Formel $—C_nH_{2n}—$ bedeutet, worin n 2 oder 3 ist,

Z für gleiche oder verschiedene Reste der Formeln —OH oder $—O—CO—CH_2—CH(SO_3M)—COOM$, steht, worin M ein Kation bedeutet, wobei mindestens ein Rest Z von —OH verschieden ist,

x eine Zahl von 1 bis 100 ist und

m eine ganze Zahl von 1 bis 5 bedeutet.

2. Verbindungen nach Anspruch 1, dadurch gekennzeichnet, daß X für $—CH_2—CH_2—$ steht.

3. Verbindungen nach Anspruch 1 oder 2, dadurch gekennzeichnet, daß Y für $—CH_2—CH_2—$ oder $—CH_2—CH(CH_3)—$ steht.

4. Verbindungen nach Anspruch 1 bis 3, dadurch gekennzeichnet, daß Z für $—O—CO—CH_2—CH(SO_3M)—COOM$ steht, wobei M die in Anspruch 1 genannte Bedeutung hat.

5. Verbindungen nach Anspruch 1 bis 4, dadurch gekennzeichnet, daß M Wasserstoff, ein Alkalimetall, Erdalkalimetall, Ammonium oder eine sich von einem niedermolekularen Alkyl- oder Alkylolamin ableitende Ammoniumgruppe bedeutet.

6. Verbindungen nach Anspruch 1 bis 5, dadurch gekennzeichnet, daß x für 2 bis 18 steht.

7. Verbindungen nach Anspruch 1 bis 5, dadurch gekennzeichnet, daß m 2 oder 3 ist, wobei die Gruppen $(X—O)_x—Y—Z$ gliech oder verschieden sind.

8. Verwendung der Verbindungen nach Anspruch 1 als grenzflächenaktive Mittel.

9. Verwendung der Verbindungen nach Anspruch 1 als Dispergier-, Netz-, Emulgier- und Färbereihilfsmittel.

**Claims**

1. Compounds of the formula

$$A[(X—O)_x—Y—Z]_m$$

wherein

A stands for the radical of a natural resinic acid, the radical of a disproportionation of hydrogenation product thereof, the radical of a resinic acid maleate the radical of a resinic acid modified by reaction with a cycloaliphatic, araliphatic or aromatic compound splitting off halogen or with a phenolic compound, or of an esterification product of a resinic acid which is optionally modified as hereinbefore defined with a polyhydric alcohol,

X stands for the same or different groups of the formula $—CH_2—CH_2—$ and $—CH_2—CH(CH_3)—$,

Y means the same or different groups of the formula $—C_nH_{2n}—$, wherein n is 2 or 3,

Z stands for the same or different radicals of the formulae —OH or $—O—CO—CH_2—CH(SO_3M)—COOM$, wherein M stands for a cation with the proviso that at least one radical Z is different from —OH,

x is a number of 1 to 100 and

m is an integer of 1 to 5.

2. Compounds according to claim 1, characterized in that X stands for $—CH_2CH_2—$.

3. Compounds according to claim 1 and 2, characterized in that Y stands for $—CH_2—CH_2—$ or $—CH_2—CH(CH_3)—$.

4. Compounds according to claims 1 to 3, characterized in that Z stands for

—O—CO—$CH_2$—CH($SO_3$M)—COOM, wherein M has the meaning mentioned in claim 1.

5. Compounds according to claims 1 to 4, characterized in that M designates hydrogen, an alkali metal, an alkaline earth metal, ammonium or an ammonium group deriving from a low-molecular alkyl or alkylol amine.

6. Compounds according to claims 1 to 5, characterized in that x stand for 2 to 18.

7. Compounds according to claims 1 to 5, characterized in that m is 2 or 3, the groups $(X—O)_x$—Y—Z being the same or different.

8. Application of the compounds according to claim 1 as interface-active agents.

9. Application of the compounds according to claim 1 as dispersing, wetting or emulsifying agent or as dyeing auxiliaries.

**Revendications**

1. Composés répondant à la formule :

$$A[(X—O)_x—Y—Z]_m$$

dans laquelle

A représente le radical d'un acide résinique naturel ou d'un de ses produits de dismutation ou d'hydrogénation, d'un acide résinique/maléate (résine abiétomaléique), d'un acide résinique modifié par réaction avec un composé cyclo-aliphatique, araliphatique ou aromatique donneur d'halogène ou avec un composé phénolique, ou d'un produit d'estérification, avec un polyol, d'un acide résinique éventuellement modifié tel qu'on vient de le définir,

X représente, ou les X représentent chacun indépendamment les uns des autres, un radical —$CH_2$—$CH_2$— ou —$CH_2$—CH($CH_3$)—,

Y représente, ou les Y représentent chacun indépendamment les uns des autres, un radical —$C_nH_{2n}$— dans lequel n est égal à 2 ou à 3,

Z représente, ou les Z représentent chacun indépendamment les uns des autres, un groupe —OH ou un radical —O—CO—$CH_2$—CH($SO_3$M)—COOM dans lequel M désigne un cation, l'un au moins des symboles Z n'étant pas un groupe —OH,

x désigne un nombre de 1 à 100 et

m désigne un nombre entier de 1 à 5.

2. Composés selon la revendication 1, caractérisés en ce que X représente —$CH_2$—$CH_2$—.

3. Composés selon l'une des revendications 1 et 2, caractérisés en ce que Y représente —$CH_2$—$CH_2$— ou —$CH_2$—CH($CH_3$)—.

4. Composés selon l'une quelconque des revendications 1 à 3, caractérisés en ce que Z représente un radical —O—CO—$CH_2$—CH($SO_3$M)—COOM dans lequel M a la signification donnée à la revendication 1.

5. Composés selon l'une quelconque des revendications 1 à 4, caractérisés en ce que M représente l'hydrogène, un métal alcalin, un métal alcalino-terreux, le groupe ammonium ou un groupe ammonium dérivant d'une alkylamine ou d'une alkylolamine à bas poids moléculaire.

6. Composés selon l'une quelconque des revendications 1 à 5, caractérisés en ce que x est un nombre de 2 à 18.

7. Composés selon l'une quelconque des revendications 1 à 5, caractérisés en ce que m est égal à 2 ou à 3 et les radicaux $(X—O)_x$—Y—Z sont identiques ou différents.

8. Application des composés selon la revendication 1 comme agents surfactifs.

9. Application des composés selon la revendication 1 comme dispersants, mouillants, émulsionnants et adjuvants de teinturerie.